# EUROPÄISCHE PATENTSCHRIFT

(11) **EP 1 793 229 B1**
(45) Veröffentlichungstag und Bekanntmachung des Hinweises auf die Patenterteilung: **08.09.2010**
(21) Anmeldenummer: 06124975.1
(22) Anmeldetag: 29.11.2006
(51) Int. Cl.: G01N 33/487, A61B 5/00

(54) **Verfahren zum akustischen Ausgeben einer Information in einem Analysesystem**
Method to give acoustically an information in an analytical system
Méthode pour donner acoustiquement une information dans un analyseur

(30) Priorität: 05.12.2005 EP 05026525
(43) Veröffentlichungstag der Anmeldung: 06.06.2007
(73) Patentinhaber: Roche Diagnostics GmbH, 68305 Mannheim (DE); F. Hoffmann-La Roche AG, 4070 Basel (CH)
(72) Erfinder: Werner, Karl, 69168 Wiesloch (DE); Albrecht, Gertrud, 68239 Mannheim (DE)
(74) Vertreter: Stößel, Matthias

(56) Entgegenhaltungen:
- EP-A- 1 256 798
- EP-A- 1 498 719
- WO-A-03/082091
- US-A- 4 014 016
- US-A1- 2004 106 859
- PATENT ABSTRACTS OF JAPAN Bd. 016, Nr. 143 (P-1335), 9. April 1992 (1992-04-09) & JP 04 001570 A (HITACHI LTD), 7. Januar 1992 (1992-01-07)

## Beschreibung

Die Erfindung bezieht sich auf ein Verfahren zum akustischen Ausgeben einer Information durch einen akustischen Signalgeber, wobei der Signalgeber in einem Analysesystem zur Analyse einer Probe mittels eines Testelements angeordnet ist und sie bezieht sich auf ein Analysesystem mit einem akustischen Signalgeber.

Zur Analyse von Proben, beispielsweise Körperflüssigkeiten wie Blut oder Urin, werden häufig Analysesysteme verwendet, bei denen die zu analysierenden Proben auf ein Testelement gegeben werden und gegebenenfalls mit einer oder mehreren Reagenzien in einem Testfeld auf dem Testelement reagieren, bevor sie analysiert werden. Die optische, insbesondere photometrische, und die elektrochemische Auswertung von Testelementen stellen die gebräuchlichsten Verfahren zur schnellen Bestimmung der Konzentration von Analyten in Proben dar. Photometrische und elektrochemische Auswertungen werden allgemein im Bereich der Analytik, der Umweltanalytik und vor allem im Bereich der medizinischen Diagnostik eingesetzt. Insbesondere im Bereich der Blutglukosediagnostik aus Kapillarblut besitzen Testelemente, die photometrisch oder elektrochemisch ausgewertet werden, einen großen Stellenwert.

In den letzten Jahren haben tragbare Messgeräte zur Blutzuckerbestimmung an Bedeutung gewonnen. Sie ermöglichen zu jeder Zeit mittels eines einfach zu bedienenden Messgeräts, einer hinsichtlich des Einstechschmerzes optimierten Stechhilfe und eines Testelements für den einmaligen Gebrauch, Blutzuckermesswerte zu bestimmen und damit eine genauere Insulindosierung des Patienten zur Stabilisierung seines Blutzuckerwertes vorzunehmen.

Es gibt verschiedene Formen von Testelementen. Bekannt sind z.B. im Wesentlichen quadratische Blättchen, die auch als Slides bezeichnet werden, in deren Mitte sich ein mehrschichtiges Testfeld befindet. Diagnostische Testelemente, die streifenförmig ausgebildet sind, werden als Teststreifen bezeichnet. Zur räumlichen Trennung von Detektionszone und Probenaufgabestelle eines Testelements sind im Stand der Technik kapillare Testelemente bekannt, z.B. aus WO 99/29429.

Die mittels eines Testelements in einem Analysesystem durchgeführte Analyse einer Probe führt zu einem Analyseergebnis, z.B. zu einem ermittelten Blutzuckerkonzentrationswert. Dieses Analyseergebnis wird üblicherweise auf einem optischen Anzeigefeld des Analysesystems, z.B. einer Flüssigkristallanzeige angezeigt. Für Anwender, die unter einer Sehbehinderung oder Blindheit leiden, wird zusätzlich eine akustische Ausgabe vorgesehen.

WO 03/039362 A1 bezieht sich auf ein reagenzloses Vollblut-Blutzuckermessgerät, bei dem ein Prozessor gemessene Konzentrationsergebnisse und/oder andere Informationen an ein Steuergerät kommuniziert. Das Steuergerät betreibt ein optisches Anzeigefeld zum Präsentieren der Information für einen Anwender. Zusätzlich oder alternativ zu dem optischen Anzeigefeld kann eine akustische Ausgabe der Information vorgesehen sein.

WO 02/062212 A1 betrifft ein Verwaltungssystem für das persönliche Befinden, das eine Ausgabevorrichtung umfasst, die einem Patienten eine Behandlungsempfehlung kommuniziert. Die Ausgabevorrichtung kann die Behandlungsempfehlung durch hörbare, sichtbare oder taktile Mittel übermitteln, beispielsweise durch einen Warntongeber oder eine Systemanzeige.

Zum akustischen Ausgeben einer Information dient üblicherweise ein akustischer Signalgeber oder ein Lautsprecher.

US 4,014,016 hat ein Ausgabesystem für Blinde zum Gegenstand, bei dem Informationen durch akustische Signale übermittelt werden. Dabei handelt es sich z.B. um ein Ausgabesystem eines Taschenrechners, bei dem die Ziffern eines Rechenergebnisses durch einheitlich beabstandete Tonfolgen mit einer unterschiedlichen Anzahl von Tönen derselben Frequenz augegeben werden.

DE 25 50 614 bezieht sich auf ein Verfahren zur hörbaren Ausgabe durch das Erzeugen eines codierten elektrischen Ausgangssignals, welches bezeichnend für eine wiedererlangbare Information ist. Dieses codierte elektrische Ausgangssignal wird zum Erzeugen einer vorbestimmten Anzahl von Hörtönen mit im Wesentlichen der gleichen Frequenz verwendet, wobei die Anzahl dieser Töne bezeichnend für die wiederzuerlangende Information ist. Die wiedererlangbare Information kann z.B. wenigstens eine Dezimalzahl sein, wobei die Anzahl der hörbaren Töne gleich der Dezimalzahl ist.

In EP-A 1 256 798 wird ein Messinstrument für einen Biosensor offenbart, welches auch einen Lautsprecher zur Abgabe eines Alarmsignals enthalten kann. Dieses Alarmsignal wird im Zusammenhang mit einem akustischen Ausgeben einer Fehlerinformation ausgegeben.

US 2004/0106859 A1 beschreibt die Überwachung eines Analyten. Dabei wird ein akustisches Alarmsignal bei Auftreten einer Besonderheit eines Messwerts ausgegeben. Beispielsweise kann ein derartiges Alarmsignal bei Überschreiten eines bestimmten Grenzwertes durch die gemessene Analytkonzentration vorgesehen sein.

In EP-A 1 498 719 wird ein Analysegerät beschrieben, bei dem ein akustischer Alarm vorgesehen sein kann. Weiterhin können bei dem Analysegerät bestimmte Geräusche den aktuellen Messzustand anzeigen.

In WO 03/082091 wird ein Probenmessgerät beschrieben, bei welchem ebenfalls ein akustisches Warnsignal vorgesehen sein kann. Dieses akustische Warnsignal kann z.B. ertönen, falls die Anzahl der in dem Gerät gespeicherten Teststreifen zur Neige geht oder falls eine Klappe eine zu lange Zeit geöffnet ist.

Eine akustische Ausgabe in einem Analysesystem zur Analyse einer Probe mittels eines Testelements muss besondere Anforderungen an seine Funktionsfähigkeit erfüllen. Von dem akustisch korrekt ausgegebenen Ergebnis der Analyse kann die Gesundheit oder auch das Leben des Anwenders abhängen. Beispielsweise hängt von einem gemessenen Blutglukosewert die Insulindosis ab, die einem Diabetiker verabreicht wird.

Daher ist es Aufgabe der vorliegenden Erfindung, akustische Fehlausgaben durch einen akustischen Signalgeber eines Analysesystems zur Analyse einer Probe mittels eines Testelements zu vermeiden beziehungsweise für den Anwender erkennbar zu machen.

Diese Aufgabe wird erfindungsgemäß gelöst durch ein Verfahren zum akustischen Ausgeben einer Information durch einen akustischen Signalgeber, wobei der Signalgeber in einem Analysesystem angeordnet ist und das Analysesystem eine Analyse einer flüssigen Probe mittels eines Testelements durchführt, wobei Analyseergebnisse generiert und durch eine Datenverarbeitungseinheit verarbeitet werden und wobei eine Steuereinheit des Analysesystems den Signalgeber ansteuert, so dass der Signalgeber akustisch eine Information ausgibt, wobei der Signalgeber ein akustisches Prüfsignal abgibt, um den Signalgeber auf Funktionsfähigkeit zu prüfen.

Ein Analysesystem ist in diesem Zusammenhang ein System zur Analyse einer flüssigen Probe, beispielsweise zur Analyse von Blut, Urin oder interstitieller Flüssigkeit, insbesondere ein Blutzuckermessgerät. Die Datenverarbeitungseinheit des Analysesystems empfängt die bei der Analyse einer auf dem Testelement angeordneten flüssigen Probe generierten Analyseergebnisse und verarbeitet diese. Beispielsweise berechnet sie aus den (z.B. photometrisch oder elektrochemisch) gemessenen Analyseergebnissen die Konzentration eines Analyten in der flüssigen Probe. Die in dem Analysesystem enthaltene Steuereinheit steuert u.a. den Signalgeber an, z.B. wenn über den Signalgeber die Information ausgegeben werden soll, welches durch die Datenverarbeitungseinheit verarbeitete Analyseergebnis sich aus der Analyse einer flüssigen Probe ergeben hat.

Als akustischer Signalgeber des Analysesystems kann jeder dem Fachmann geläufige akustische Signalgeber vorliegen, z.B. ein Summer, ein Lautsprecher oder ein piezoelektrischer Signalgeber.

Die ausgegebene Information kann z.B. eine Information zu dem Betriebsstatus des Analysesystems oder zu einem Analyseergebnis sein.

Erfindungsgemäß gibt der Signalgeber in dem Analysesystem ein akustisches Prüfsignal ab, um die Funktionsfähigkeit des Signalgebers zu prüfen. Der Anwender der Analysesystems kann dann erkennen, ob das akustische Prüfsignal korrekt ist oder nicht. Falls das akustische Prüfsignal von dem Signalgeber nicht korrekt abgegeben wird, ist davon auszugehen, dass eine durch den akustischen Signalgeber ausgegebene Information ebenfalls inkorrekt sein könnte.

Die Erfindung bezieht sich weiterhin auf ein Analysesystem zur Analyse einer flüssigen Probe mittels eines Testelements, enthaltend einen akustischen Signalgeber zum akustischen Ausgeben einer Information, ein Analysegerät zur Generierung von Analyseergebnissen, eine Datenverarbeitungseinheit zum Verarbeiten der Analyseergebnisse und eine Steuereinheit, wobei die Steuereinheit so ausgelegt ist, dass sie den Signalgeber zum Abgeben eines akustischen Prüfsignals ansteuert, um den Signalgeber auf Funktionsfähigkeit zu prüfen.

Gemäß einer bevorzugten Ausführungsform des erfindungsgemäßen Analysesystems enthält das Analysesystem einen Schalter, der durch das Einschieben oder Einlegen eines Testelements in das Analysesystem ausgelöst wird, wobei der Schalter so an das Steuergerät gekoppelt ist, dass das Steuergerät beim Auslösen des Schalters den Signalgeber zum Abgeben eines akustischen Prüfsignals ansteuert. Ein Testelement muss dabei manuell oder automatisch vor der Durchführung einer Analyse eine flüssigen Probe in eine Probenaufnahmeposition zur Aufnahme der flüssigen Probe auf das Testelement und/oder eine Messposition zur Durchführung einer Messung und Generierung von Analyseergebnissen in das Analysesystem eingeschoben oder eingelegt werden.

Das akustische Prüfsignal kann aber auch abgegeben werden, sobald der Benutzer des Analysesystems einen bestimmten Knopf des Analysesystems drückt (z.B. den Knopf zum Einschalten des Analysesystems) oder automatisch zu mindestens einem bestimmten Zeitpunkt während des Betriebes des Analysesystems.

Gemäß einer bevorzugten Ausführungsform der vorliegenden Erfindung gibt der Signalgeber ein akustisches Prüfsignal ab, das einen Ton oder eine Folge von Tönen umfasst, wobei der Ton oder die Folge von Tönen im Wesentlichen eine gleich bleibende Frequenz aufweist oder wobei der Ton oder die Folge von Tönen eine sich ändernde Frequenz aufweist.

Die Folge von Tönen kann dabei so abgegeben werden, dass die einzelnen Töne ineinander übergehen oder zumindest teilweise durch Pausen zwischen den Tönen unterbrochen werden.

Ferner kann der Signalgeber bei dem erfindungsgemäßen Verfahren ein akustisches Prüfsignal abgeben, das eine Folge von Tönen umfasst, wobei die Töne zumindest teilweise verschiedene Tonlängen aufweisen oder wobei die Töne im Wesentlichen die gleiche Tonlänge aufweisen.

Der akustische Signalgeber kann erfindungsgemäß auch ein akustisches Prüfsignal abgeben, das einen Ton oder eine Folge von Tönen umfasst, wobei sich die Lautstärke des Tons oder der Folge von Tönen ändert oder wobei der Ton oder die Folge von Tönen im Wesentlichen die gleiche Lautstärke aufweist.

Das akustische Prüfsignal sollte insbesondere hinsichtlich Tonfrequenzen, Tonlängen, Tonlautstärken, Frequenz- und Lautstärkeänderungen möglichst alle Merkmale aufweisen, die ein von dem Signalgeber im normalen Betrieb des Analysesystems abgegebenes akustisches Signal zum Ausgeben einer Information aufweisen kann. Falls ein solches akustisches Signal zum Ausgeben einer Information lediglich aus einer Folge von Tönen im Wesentlichen derselben Frequenz, Tonlänge und Lautstärke besteht, wobei die einzelnen Töne durch Pausen unterbrochen werden, so sollte das Prüfsignal zumindest dieselben Merkmale aufweisen, um eine Fehlfunktion sicher feststellen zu können. Ferner ist das Prüfsignal so gestaltet, dass ein Anwender das korrekte Prüfsignal und eventuelle Abweichungen davon leicht erkennen kann.

Gemäß einer bevorzugten Ausführungsform der vorliegenden Erfindung gibt der Signalgeber das Prüfsignal zu Beginn einer Inbetriebnahme des Analysesystems ab. Der Signalgeber kann aber auch zu jedem anderen Zeitpunkt des Betriebs des Analysesystems das oder mindestens ein zusätzliches Prüfsignal abgeben. Der Vorteil einer Abgabe des Prüfsignals zu Beginn einer Inbetriebnahme des Analysesystems ist, dass der Anwender von der Durchführung einer Analyse absehen kann, wenn er gleich zu Beginn eine Fehlfunktion des akustischen Signalgebers des Analysesystems feststellt. Damit können z.B. nutzlose schmerzhafte Blutentnahmen oder eine Verschwendung von Testelementen für Analysen mit dem Analysesystem vermieden werden, die aufgrund der Fehlfunktion des akustischen Signalgebers nicht verwertbar sind. Der Anwender kann bei Erkennen eines fehlerhaften Prüfsignals aber auch einen nicht sehbehinderten Betreuer um ein Ablesen der Information auf einer optischen Anzeige bitten, falls eine solche optische Anzeige vorhanden ist.

Die Abgabe des Prüfsignals gleich zu Beginn der Inbetriebnahme des Analysesystems kann beispielsweise durch das Einschalten des Analysesystems oder durch das Einschieben eines Testelements automatisch oder durch den Anwender in das Analysesystem ausgelöst werden. Die Überprüfung der Funktionsfähigkeit des Signalgebers mittels des akustischen Prüfsignals kann gleichzeitig mit der Überprüfung der Funktionsfähigkeit einer optischen Anzeige des Analysesystems, z.B. einer Flüssigkristallanzeige, erfolgen, so dass keine zusätzliche Verzögerung bis zur Einsatzbereitschaft des Analysesystems in Kauf genommen werden muss.

Gemäß einer bevorzugten Ausführungsform der vorliegenden Erfindung codiert der akustische Signalgeber eine Information akustisch, die als Zahl mit mindestens einer Ziffer darstellbar ist. Die Zahl kann z.B. eine mit dem Analysesystem ermittelte Konzentration eines Analyten in einer auf einem Testelement analysierten Probe sein, insbesondere die Glukosekonzentration in Blut. Die Zahl kann aus einer oder mehreren Ziffern bestehen und gegebenenfalls Nachkommastellen aufweisen.

Zusätzlich oder alternativ dazu kann die durch den akustischen Signalgeber codierte Information eine Information bezüglich der Betriebsbereitschaft des Analysesystems (z.B. dass das Analysesystem eingeschaltet wurde) oder eine Information über den Zeitpunkt zur Durchführung einer bestimmten Handlung durch den Anwender (z.B. den Zeitpunkt zum Anlegen seines Fingers an eine in das Analysesystem integrierte Stechhilfe oder den Zeitpunkt zum Übertragen einer zu analysierenden Probe auf ein Testelement) umfassen. Der Signalgeber kann auch akustisch eine Information bezüglich der Notwendigkeit zum Auswechseln oder Nachfüllen eines in dem Analysesystem verwendeten Verbrauchsmittels (z.B. Testelement, Testelementmagazin, Batterie, Lanzette oder Disposable) ausgeben.

Gemäß einer bevorzugten Ausführungsform der vorliegenden Erfindung codiert der akustische Signalgeber eine Information als Folge von Tönen akustisch, wobei die Anzahl der Töne bezeichnend für die Information ist. Eine Zahl kann beispielsweise so akustisch ausgegeben werden, dass eine Anzahl von hörbaren Ausgangstönen jeweils einem Ziffernwert für jede Ziffer der Zahl entspricht, wobei die Ausgabe jeweils einer neuen Ziffer z.B. durch eine längere Pause oder ein anderes akustisches Signal ausgegeben wird. Ferner kann ein bestimmtes akustisches Signal für einen Dezimalpunkt und/oder ein Vorzeichen vorgesehen sein.

Des Weiteren bezieht sich die Erfindung auf die Verwendung eines akustischen Signalgebers, der zum akustischen Ausgeben einer Information in einem Analysesystem zur Analyse einer Probe mittels eines Testelements angeordnet ist, zur Überprüfung der Funktionsfähigkeit des Signalgebers durch eine Abgabe eines akustischen Prüfsignals.

## Patentansprüche

1. Verfahren zum akustischen Ausgeben einer Information durch einen akustischen Signalgeber, wobei der Signalgeber in einem Analysesystem angeordnet ist und das Analysesystem eine Analyse einer flüssigen Probe mittels eines Testelements durchführt, wobei Analyseergebnisse generiert und durch eine Datenverarbeitungseinheit verarbeitet werden und wobei eine Steuereinheit des Analysesystems den Signalgeber ansteuert, so dass der Signalgeber akustisch eine Information ausgibt, **dadurch gekennzeichnet, dass** der Signalgeber ein akustisches Prüfsignal abgibt, um den Signalgeber auf Funktionsfähigkeit zu prüfen, wobei der Signalgeber das Prüfsignal zu Beginn einer Inbetriebnahme des Analysesystems abgibt, wobei das Prüfsignal derart gestaltet ist, dass ein Anwender das korrekte Prüfsignal und eventuelle Abweichungen davon leicht erkennen kann.

2. Verfahren gemäß Anspruch 1, **dadurch gekennzeichnet, dass** der Signalgeber ein akustisches Prüfsignal abgibt, das einen Ton oder eine Folge von Tönen umfasst, wobei der Ton oder die Folge von Tönen im Wesentlichen eine gleich bleibende Frequenz aufweist oder wobei der Ton oder die Folge von Tönen eine sich ändernde Frequenz aufweist.

3. Verfahren gemäß einem der Ansprüche 1 oder 2, **dadurch gekennzeichnet, dass** der Signalgeber ein akustisches Prüfsignal abgibt, das eine Folge von Tönen umfasst, wobei die Töne zumindest teilweise verschiedene Tonlängen aufweisen oder wobei die Töne im Wesentlichen die gleiche Tonlänge aufweisen.

4. Verfahren gemäß einem der Ansprüche 1 bis 3, **dadurch gekennzeichnet, dass** der Signalgeber ein akustisches Prüfsignal abgibt, das einen Ton oder eine Folge von Tönen umfasst, wobei sich die Lautstärke des Tons oder der Folge von Tönen ändert oder wobei der Ton oder die Folge von Tönen im Wesentlichen die gleiche Lautstärke aufweist.

5. Verfahren gemäß einem der Ansprüche 1 bis 4, **dadurch gekennzeichnet, dass** der akustische Signalgeber eine Information akustisch codiert, die als Zahl mit mindestens einer Ziffer darstellbar ist.

6. Verfahren gemäß einem der Ansprüche 1 bis 5, **dadurch gekennzeichnet, dass** der akustische Signalgeber eine Information als Folge von Tönen akustisch codiert, wobei die Anzahl der Töne bezeichnend für die Information ist.

7. Analysesystem zur Analyse einer flüssigen Probe mittels eines Testelements, enthaltend einen akustischen Signalgeber zum akustischen Ausgeben einer Information, ein Analysegerät zur Generierung von Analyseergebnissen, eine Datenverarbeitungseinheit zum Verarbeiten der Analyseergebnisse und eine Steuereinheit, **dadurch gekennzeichnet, dass** die Steuereinheit so ausgelegt ist, dass sie den Signalgeber zum Abgeben eines akustischen Prüfsignals ansteuert, um den Signalgeber auf Funktionsfähigkeit zu prüfen, wobei der Signalgeber das Prüfsignal zu Beginn einer Inbetriebnahme des Analysesystems abgibt.

8. Analysesystem gemäß Anspruch 7, **dadurch gekennzeichnet, dass** das Analysesystem einen Schalter enthält, der durch das Einschieben eines Testelements in das Analysesystem ausgelöst wird, wobei der Schalter so an das Steuergerät gekoppelt ist, dass das Steuergerät beim Auslösen des Schalters den Signalgeber zum Abgeben eines akustischen Prüfsignals ansteuert.

9. Verwendung eines akustischen Signalgebers, der zum akustischen Ausgeben einer Information in einem Analysesystem zur Analyse einer Probe mittels eines Testelements angeordnet ist, zur Überprüfung der Funktionsfähigkeit des Signalgebers durch eine Abgabe eines akustischen Prüfsignals, wobei der Signalgeber das Prüfsignal zu Beginn einer Inbetriebnahme des Analysesystems abgibt.

## Claims

1. Method for the audible output of a piece of information by an audible signal generator, where the signal generator is arranged in an analysis system and the analysis system analyses a fluid sample using a test element, where analysis results are generated and are processed by a data processing unit, and where a control unit in the analysis system actuates the signal generator, so that the signal generator audibly outputs a piece of information, **characterized in that** the signal generator sends an audible test signal in order to test the operability of the signal generator, where the signal generator sends the test signal at the start of the analysis system's being started up, where the test signal is designed such that a user can easily identify the correct test signal and any deviations therefrom.

2. Method according to Claim 1, **characterized in that** the signal generator sends an audible test signal which comprises a tone or a sequence of tones, the tone or the sequence of tones essentially having a constant frequency or the tone or the sequence of tones having a changing frequency.

3. Method according to either of Claims 1 and 2, **characterized in that** the signal generator sends an audible test signal which comprises a sequence of tones, at least some of the tones having different tone lengths or the tones having essentially the same tone length.

4. Method according to one of Claims 1 to 3, **characterized in that** the signal generator sends an audible test signal which comprises a tone or a sequence of tones, the volume of the tone or of the sequence of tones changing or the tone or the sequence of tones having essentially the same volume.

5. Method according to one of Claims 1 to 4, **characterized in that** the audible signal generator audibly encodes a piece of information which can be represented as a number with at least one digit.

6. Method according to one of Claims 1 to 5, **characterized in that** the audible signal generator audibly encodes a piece of information as a sequence of tones, the number of tones denoting the piece of information.

7. Analysis system for analysing a fluid sample using a test element, containing an audible signal generator for audibly outputting a piece of information, an analyser for generating analysis results, a data processing unit for processing the analysis results and a control unit, **characterized in that** the control unit is designed such that it actuates the signal generator to send an audible test signal in order to test the operability of the signal generator, where the signal generator sends the test signal at the start of the analysis system's being started up.

8. Analysis system according to Claim 7, **characterized in that** the analysis system contains a switch which is tripped by a test element's being pushed into the analysis system, the switch being coupled to the controller such that the controller actuates the signal generator to send an audible test signal when the switch is tripped.

9. Use of an audible signal generator, which, for the purpose of audibly outputting a piece of information, is arranged in an analysis system for analysing a sample using a test element, for checking the operability of the signal generator by sending an audible test signal, where the signal generator sends the test signal at the start of the analysis system's being started up.

## Revendications

1. Procédé de diffusion sonore d'une information par un générateur de signal sonore, le générateur de signal étant disposé dans un système d'analyse et le système d'analyse effectuant une analyse d'un échantillon liquide au moyen d'un élément de test, des résultats d'analyse étant générés et traités par une unité de traitement de données et une unité de contrôle du système d'analyse commandant le générateur de signal de telle sorte que le générateur de signal délivre une information sous forme sonore, **caractérisé en ce que** le générateur de signal délivre un signal de contrôle sonore pour vérifier l'aptitude fonctionnelle du générateur de signal, le générateur de signal délivrant le signal de contrôle au début d'une mise en service du système d'analyse, le signal de contrôle étant configuré de telle sorte qu'un utilisateur peut facilement reconnaître le signal de contrôle correct et les éventuelles divergences par rapport à celui-ci.

2. Procédé selon la revendication 1, **caractérisé en ce que** le générateur de signal délivre un signal de contrôle sonore qui comprend une tonalité ou une séquence de tonalités, la tonalité ou la séquence de tonalités présentant pour l'essentiel une fréquence constante ou la tonalité ou la séquence de tonalités présentant une fréquence qui varie.

3. Procédé selon l'une des revendications 1 ou 2, **caractérisé en ce que** le générateur de signal délivre un signal de contrôle sonore qui comprend une séquence de tonalités, les tonalités présentant au moins en partie des longueurs de tonalité différentes ou les tonalités présentant pour l'essentiel la même longueur de tonalité.

4. Procédé selon l'une des revendications 1 à 3, **caractérisé en ce que** le générateur de signal délivre un signal de contrôle sonore qui comprend une tonalité ou une séquence de tonalités, le volume sonore de la tonalité ou de la séquence de tonalités changeant ou la tonalité ou la séquence de tonalités présentant pour l'essentiel le même volume sonore.

5. Procédé selon l'une des revendications 1 à 4, **caractérisé en ce que** le générateur de signal sonore effectue le codage sonore d'une information qui peut être représentée sous la forme d'un nombre à au moins un chiffre.

6. Procédé selon l'une des revendications 1 à 5, **caractérisé en ce que** le générateur de signal sonore effectue le codage sonore d'une information sous la forme d'une séquence de tonalités, le nombre de tonalités identifiant l'information.

7. Système d'analyse pour analyser un échantillon liquide au moyen d'un élément de test, comprenant un générateur de signal sonore pour la diffusion sonore d'une information, un analyseur pour générer des résultats d'analyse, une unité de traitement de données pour traiter les résultats d'analyse et une unité de contrôle, **caractérisé en ce que** l'unité de contrôle est conçue de telle sorte qu'elle commande le générateur de signal pour qu'il délivre un signal de contrôle sonore afin de vérifier l'aptitude fonctionnelle du générateur de signal, le générateur de signal délivrant le signal de contrôle au début d'une mise en service du système d'analyse.

8. Système d'analyse selon la revendication 7, **caractérisé en ce que** le système d'analyse contient un commutateur qui est déclenché par l'introduction d'un élément de test dans le système d'analyse, le commutateur étant couplé au module de contrôle de telle sorte que le module de contrôle, lors du déclenchement du commutateur, commande le générateur de signal pour qu'il délivre un signal de contrôle sonore.

9. Utilisation d'un générateur de signal sonore qui, pour la diffusion sonore d'une information, est disposé dans un système d'analyse pour analyser un échantillon au moyen d'un élément de test, pour contrôler l'aptitude fonctionnelle du générateur de signal en délivrant un signal de contrôle sonore, le générateur de signal délivrant le signal de contrôle au début d'une mise en service du système d'analyse.
